# NOUVEAU FASCICULE DE BREVET EUROPEEN

(11) **EP 0 758 545 B2**
(45) Date de publication et mention de la décision concernant l'opposition: **17.11.2004**
(45) Mention de la délivrance du brevet: 18.07.2001
(21) Numéro de dépôt: 96401627.3
(22) Date de dépôt: 19.07.1996
(51) Int. Cl.: A61K 7/06

(54) **Composition cosmétique contenant une dispersion aqueuse de polymère et une silicone insoluble, utilisation et procédé**
Kosmetische Zusammensetzung die eine wässrige Polymerdispersion und ein unlösliches Siliconderivat enthält, seine Verwendung und Herstellung
Cosmetic composition containing an aqueous polymer dispersion and an insoluble silicone, its utilization and preparation

(30) Priorité: 11.08.1995 FR 9509773
(43) Date de publication de la demande: 19.02.1997
(73) Titulaire: L'OREAL, 75008 Paris (FR)
(72) Inventeur: Samain, Henri, 91570 Bievres (FR); Bauer, Daniel, 93340 Le Raincy (FR); Sturla, Jean-Michel, 92210 Saint Cloud (FR)
(74) Mandataire: Andral, Christophe André Louis

(56) Documents cités:
- EP-A- 0 424 260
- EP-A- 0 590 604
- EP-A- 0 635 257
- WO-A-92/21316
- US-A- 5 314 684

## Description

L'invention concerne une composition cosmétique aqueuse ou hydroalcoolique contenant une dispersion aqueuse de particules insolubles de polymère filmogène et une silicone insoluble, les particules de polymères étant présentes à une concentration supérieure à 15 % en poids. L'invention concerne également un procédé de traitement cosmétique à l'aide de ces compositions, ainsi que l'utilisation d'une silicone insoluble pour améliorer la qualité de la pulvérisation d'une composition cosmétique contenant une dispersion aqueuse de particules insolubles de polymère filmogène.

Les compositions capillaires à pulvériser sur les cheveux sont essentiellement constituées d'une solution le plus souvent hydroalcoolique et d'un polymère en mélange éventuellement avec divers adjuvants cosmétiques. Cette solution est conditionnée soit dans un flacon pompe, soit dans un récipient aérosol approprié qui est mis sous pression à l'aide d'un gaz propulseur.

Depuis quelques années, un intérêt tout particulier s'est manifesté pour la réalisation de compositions cosmétiques capillaires essentiellement aqueuses. En effet, l'emploi d'alcool tel que l'éthanol ou l'isopropanol, seul ou en mélange avec une faible proportion d'eau, peut présenter certains inconvénients, notamment une augmentation de l'inflammabilité lorsque la composition est sous forme d'une laque aérosol.

De manière encore plus générale, on cherche à réduire l'emploi des composés volatils à la pression atmosphérique, dits COV (Composés Organiques Volatils), qui sont présents dans les compositions cosmétiques. Les COV sont principalement les propulseurs et certains solvants tels que l'éthanol.

Pour diminuer la quantité de COV, on a essayé de remplacer les solvants tels que l'éthanol par de l'eau. Toutefois, si la plupart des polymères filmogènes hydrosolubles peuvent, en solution dans l'eau, conduire à l'obtention de compositions de fixation des cheveux, ces dernières présentent des inconvénients majeurs. Ainsi, les compositions essentiellement aqueuses de ces polymères ne permettent pas d'obtenir de hauts degrés de fixation. Il a certes été proposé d'utiliser ces polymères hydrosolubles à des concentrations élevées, mais l'augmentation de concentration provoque un tel accroissement de la viscosité des compositions que l'on ne peut que très difficilement obtenir une pulvérisation satisfaisante. Même si une pulvérisation correcte est obtenue, ces compositions aqueuses présentent un temps de séchage particulièrement long par rapport aux compositions alcooliques, et sont donc d'un faible intérêt pratique.

Il a également été proposé d'utiliser des dispersions aqueuses de particules insolubles de polymères à la place des polymères solubilisés dans des compositions aqueuses, alcooliques ou hydroalcooliques. Cependant, jusqu'à présent, les résultats obtenus ne sont pas encore satisfaisants. En particulier, la demanderesse a constaté que lorsqu'on cherche à mettre en oeuvre des concentrations élevées de particules de polymère en dispersion aqueuse, la pulvérisation du spray n'est pas satisfaisante. Les particules liquides pulvérisées ne sont pas fines, le spray est souvent pincé, c'est à dire non diffus et la pulvérisation est irrégulière. De plus, l'orifice de pulvérisation a tendance à se boucher.
La pulvérisation étant un élément essentiel pour la qualité finale d'une composition à pulvériser sur les cheveux, il est primordial de remédier à ces inconvénients pour obtenir une bonne répartition du spray sur l'ensemble de la chevelure.

La demanderesse a maintenant découvert qu'une composition cosmétique contenant, dans un milieu cosmétiquement acceptable, une dispersion aqueuse de particules de polymères insolubles et une silicone insoluble de poids moléculaire moyen variant entre 1000 et 1 000 000, les particules de polymères étant présentes à une concentration supérieure à 15 % en poids et la température de transition vitreuse de l'extrait sec de la composition étant comprise entre 15 et 35°C, permettait de remédier aux inconvénients décrits ci-dessus, c'est à dire pouvait être facilement et correctement pulvérisée.

Les compositions selon l'invention permettent d'obtenir une bonne pulvérisation, le spray est régulier et les gouttes pulvérisées sont fines. Les compositions se répartissent facilement sur l'ensemble de la chevelure. De plus, de façon surprenante, le pouvoir plixant des compositions n'est pas diminué par l'addition d'une silicone. Enfin, les temps de séchage sont faibles.

La présente invention a donc pour objet une composition cosmétique contenant, dans un milieu cosmétiquement acceptable aqueux ou hydroalcoolique, une dispersion aqueuse de particules insolubles de polymère et une silicone insoluble de poids moléculaire moyen variant entre 1000 et 1 000 000, les particules de polymères étant présentes à une concentration supérieure à 15 % en poids par rapport au poids total de la composition et la température de transition vitreuse de l'extrait sec de la composition étant comprise entre 15 et 35°C.

L'invention concerne également l'utilisation d'une silicone insoluble de poids moléculaire moyen variant entre 1000 et 1 000 000 (ie. Insoluble dans un milieu aqueux ou hydroalcoolique) pour améliorer la qualité de la vaporisation/ pulvérisation d'une composition cosmétique contenant dans un milieu aqueux ou hydroalcoolique cosmétiquement acceptable une dispersion aqueuse de particules insolubles de polymère, les particules de polymères étant présentes à une concentration supérieure à 15 % en poids par rapport au poids total de la composition, la température de transition vitreuse de l'extrait sec de la composition étant comprise entre 15 et 35°C.

Les compositions selon l'invention présentent, outre les avantages précités, un bon pouvoir plixant, une bonne résistance à l'humidité, une bonne élimination au shampooing et au brossage et une bonne vitesse de séchage.

Mais d'autre caractéristiques, aspects ou avantages de l'invention apparaîtront encore plus complètement à la lecture de la description détaillée qui va suivre et des exemples concrets.

Les dispersions aqueuses de particules insolubles de polymère utilisables selon l'invention sont généralement obtenues par polymérisation ou copolymérisation en suspension ou en émulsion de monomères selon les procédés bien connus de l'état de la technique ( de telles dispersions sont aussi connues sous le nom de "latex").

Les dispersions aqueuses peuvent résulter en particulier de la polymérisation ou de la copolymérisation de monomères tels que le styrène, le butadiène, l'éthylène, le propylène, le vinyl toluène, le vinyl propionate, l'alcool vinylique, l'acrylonitrile, le chloroprène, l'acétate de vinyle, les uréthannes, l'isoprène, l'isobutène, l'éther vinylique, la vinylpyrrolidone, le vinylimidazole et les acides acrylique ou méthacrylique, maléïque, crotonique ou itaconique, leurs esters ou leurs amides.

Selon l'invention, on peut par exemple utiliser une dispersion aqueuse comprenant un copolymère acrylique formé de (a) environ 35 à 75% en poids d'un acrylate d'alkyle, (b) environ 25 à 65% de méthacrylate d'alkyle et (c) environ 1 à 15% d'un ou plusieurs acides carboxyliques éthyléniques ayant de 3 à 5 atomes de carbone, les radicaux alkyle ayant de 1 à 5 atomes de carbone, ces pourcentages étant exprimés en poids par rapport au poids total de copolymère.

L'acrylate d'alkyle est de préférence choisi parmi l'acrylate de méthyle, l'acrylate d'éthyle, l'acrylate de propyle et l'acrylate de butyle. L'acrylate d'éthyle est particulièrement préféré.
La concentration en acrylate d'alkyle est de préférence comprise entre 40 et 70% en poids et plus particulièrement entre 50 et 60% en poids par rapport au poids total du copolymère.

Le méthacrylate d'alkyle est de préférence choisi parmi le méthacrylate de méthyle, le méthacrylate d'éthyle, le méthacrylate de propyle et le méthacrylate de butyle. Le méthacrylate de méthyle est particulièrement préféré.
La concentration en méthacrylate d'alkyle est de préférence comprise entre 30 et 50% en poids et plus particulièrement entre 30 et 40% en poids par rapport au poids total du copolymère.

Les acides carboxyliques éthyléniques préférés sont l'acide acrylique, l'acide méthacrylique, l'acide crotonique, l'acide itaconique ou leurs mélanges. L'acide acrylique et l'acide méthacrylique sont particulièrement préférés. Selon l'invention, il est possible de mettre en oeuvre des sels de ces acides carboxyliques.

La concentration en acides carboxyliques éthyléniques, ou en leurs sels, est de préférence comprise entre 5 et 15% en poids et plus particulièrement entre 8 et 12% en poids par rapport au poids total du copolymère.

Dans un mode de réalisation particulièrement préféré de l'invention, l'acide acrylique est utilisé avec l'acide méthacrylique, chacun dans une concentration comprise entre 2 et 10% en poids, le total de ces deux acides n'excédant pas 15% en poids du poids total du copolymère.

Le copolymère peut également contenir des faibles quantités, c'est à dire moins de 10%, de préférence moins de 5% et plus particulièrement moins de 2%, d'un monomère polymérisable autre que ceux mentionnés ci-avant.

Généralement, la dispersion contient au moins 0,5% de tensioactif permettant la mise en dispersion et le maintien en dispersion du polymère insoluble. Selon l'invention, on peut utiliser tout type de tensioactif, mais de préférence un tensioactif non ionique, et plus particulièrement les alkyl(C₆-C₁₂)phénols polyoxyalkylénés.

La taille moyenne des particules du copolymère dans la dispersion est de préférence comprise entre 0,1 et 1 micron.

Selon un mode particulièrement préféré de mise en oeuvre de l'invention, on utilise un copolymère comprenant de 50 à 60% en poids d'acrylate d'éthyle, de 30 à 40% en poids de méthacrylate de méthyle, de 2 à 10% en poids d'acide acrylique, de 2 à 10% en poids d'acide méthacrylique, la concentration totale d'acide acrylique et méthacrylique n'excédant pas 15% en poids par rapport au poids total du copolymère acrylique.

Un tel copolymère est par exemple décrit dans la demande de brevet EP-A-590604.

Une dispersion aqueuse du copolymère acrylique décrit ci-dessus comprenant 25% en poids d'un copolymère acrylate d'éthyle / méthacrylate de méthyle/ acide méthacrylique/ acide acrylique ayant une température de transition vitreuse d'environ 30°C, est vendu notamment sous la dénomination commerciale AMERHOLD DR-25 par la société AMERCHOL.

Des dispersions aqueuses convenant particulièrement bien à l'invention sont aussi les dispersions aqueuses de copolymères styrène/acrylate de butyle tels que par exemple le produit vendu sous la dénomination commerciale URAMUL SC 70 par la société D.S.M. Resins.

La concentration en poids des particules de polymère insoluble dans les compositions selon l'invention est de préférence comprise entre 15 et 35% par rapport au poids total de la composition.

Les silicones insolubles utilisables dans le cadre de la présente invention peuvent être choisis parmi toutes celles déjà connues en soi, à savoir notamment celles décrites dans les demandes de brevets EP-A- 0 181773 et EP-A- 0 473508.

Il est bien entendu possible de mettre en oeuvre des mélanges de silicones. Par silicone insoluble, on entend une silicone insoluble dans le milieu servant de support aux compositions selon l'invention.

Ainsi, selon la présente invention, il est possible d'utiliser toute silicone connue en soi, qu'il s'agisse d'une huile, d'une résine ou bien encore d'une gomme de silicone. Les silicones sont des polymères ou oligomères organo-siliciés à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane), des radicaux hydrocarbonés éventuellement substitués étant directement liés par l'intermédiaire d'un atome de carbone sur lesdits atomes de silicium. Les radicaux hydrocarbonés les plus courants sont les radicaux alkyles et en particulier méthyle, les radicaux fluoroalkyles, les radicaux aryles et en particulier phényle, et les radicaux alcényles et en particulier vinyle; d'autres types de radicaux susceptibles d'être liés soit directement, soit par l'intermédiaire d'un radical hydrocarboné, à la chaîne siloxanique sont notamment l'hydrogène, les halogènes et en particulier le chlore, le brome ou le fluor, les thiols, les radicaux alcoxy, les radicaux polyoxyalkylènes (ou polyéthers) et en particulier polyoxyéthylène et/ou polyoxypropylène, les radicaux hydroxyles ou hydroxyalkyles, les groupements aminés substitués ou non, les groupements amides, les radicaux acyloxy ou acyloxyalkyles, les radicaux hydroxyalcylamino ou aminoalkyles, des groupements ammonium quaternaires, des groupements amphotères ou bétaïniques, des groupements anioniques tels que carboxylates, thioglycolates, sulfosuccinates, thiosulfates, phosphates et sulfates, cette liste n'étant bien entendu nullement limitative (silicones dites "organomodifiées"). D'une manière générale, les silicones utilisables dans le cadre de la présente invention sont celles qui sont notamment décrites dans "Encyclopedia of Chemical Technology, Kirk-Othmer, Third Edition, 1982, volume 20, pp. 922 et suivantes" et dans "Chemistry and Technology of Silicones, Walter NOLL, Academic Press lnc, San Diego California, 1968". Le poids moléculaire moyen des silicones utilisables selon l'invention varie entre 1000 et 1 000 000. Selon la présente invention, on peut bien entendu soit utiliser une seule et même silicone soit, mettre en oeuvre plusleurs silicones différentes.

A titre d'exemples de silicones utilisables dans les compositions selon l'invention, on peut notamment citer les polydialkylsiloxanes, les polyalkylarylsiloxanes, les polydiaryldialkylsiloxanes et d'une manière encore plus générale tous les polyalkylarylsiloxanes décrits dans la demande de brevet publiée sous le numéro WO 93/05762.

Selon un mode de réalisation particulièrement préféré de la présente invention, les silicones utilisées sont choisies parmi les diorganopolysiloxanes (huiles, gommes ou résines), de préférence les polydialkylsiloxanes ou les polyalkylarylsiloxanes, et encore plus préférentiellement les polydiméthylsiloxanes éventuellement modifiés.

Les gommes de silicone sont particulièrement préférées et en particulier celles de polydialkylsiloxanes ou de polyalkylarylsiloxanes, éventuellement modifiés. Elles peuvent être utilisées seules ou en mélange dans un solvant choisi par exemple parmi les silicones volatiles, les huiles polydiméthylsiloxane ou polyphénylméthylsiloxane, les iso-paraffines, le pentane, le dodécane ou leurs mélanges.

La ou les silicones sont présentes dans les compositions conformes à l'invention dans des proportions généralement comprises entre 0,05 à 10 % en poids, de préférence de 0,1 à 3 % en poids, par rapport au poids total de la composition.

Le milieu continu aqueux ou hydroalcoolique cosmétiquement acceptable servant de support aux compositions selon l'invention, est de préférence constitué par de l'eau ou un mélange d'eau et de solvants cosmétiquement acceptables tels que des monoalcools, des polyalcools et des éthers de glycol, qui peuvent être utilisés seuls ou en mélange. Encore plus préférentiellement, ledit support est essentiellement constitué d'eau.

Le pH des compositions selon l'invention est généralement compris entre 2 et 9, et en particulier entre 3 et 8. Il peut être ajusté à la valeur désirée au moyen d'agents alcalinisants ou acidifiants habituellement utilisés en cosmétique pour ce type d'application.

Lorsque la composition selon l'invention est pressurisée sous forme d'aérosol, l'aérosol comprend la composition décrite ci-dessus, appelée jus, et au moins un agent propulseur qui peut être choisi parmi les hydrocarbures volatils tels que le n-butane, le propane, l'isobutane, le pentane, les hydrocarbures chlorés et/ou fluorés et leurs mélanges. On peut également utiliser en tant qu'agent propulseur le gaz carbonique, le protoxyde d'azote, le diméthyléther, l'azote, l'air comprimé et leurs mélanges.

Dans un tel système, la concentration en propulseur(s) est généralement comprise entre 10 et 50% en poids par rapport au poids total de la composition pressurisée et de préférence entre 15 et 35% en poids.

Les compositions selon l'invention ( à l'état pressurisé ou non) peuvent encore contenir des agents tensioactifs, des agents conservateurs, des séquestrants, des adoucissants, des parfums, des colorants, des agents modificateurs de viscosité, des agents modificateurs de mousse, des agents anti-mousse, des agents nacrants, des agents hydratants, des agents antipelliculaires, des agents antiséborrhéiques, des filtres solaires, des protéines, des vitamines, des plastifiants, des hydroxyacides, des électrolytes et des parfums.

Les compositions selon l'invention (à l'état pressurisé ou non) peuvent également contenir des agents conditionneurs. Ceux-ci peuvent alors être choisis parmi les huiles et les cires naturelles ou synthétiques, les alcools gras, les esters d'alcools polyhydriques, les glycérides, les polymères ou les mélanges de ces différents composés

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Les compositions selon l'invention sont par exemple des compositions capillaires rincées ou non rincées.

Elles sont plus particulièrement des lotions de mise en plis, des lotions pour le brushing, des compositions de fixation (laques) et de coiffage. Les lotions sont conditionnées sous diverses formes notamment dans des vaporisateurs, des flacons pompe ou dans des récipients aérosols afin d'assurer une application de la composition sous forme vaporisée.

L'invention a encore pour objet un procédé de traitement cosmétique des matières kératiniques, telles que les cheveux, caractérisé en ce qu'il consiste à appliquer sur les matières kératiniques ,en particulier par pulvérisation ou vaporisation, une composition cosmétique telle que définie précédemment, puis à effectuer éventuellement un rinçage à l'eau, après un éventuel temps de pose.

## Revendications

1. Composition cosmétique comprenant, dans un milieu cosmétiquement acceptable aqueux ou hydroalcoolique, une dispersion aqueuse de particules insolubles de polymère et une silicone insoluble, **caractérisée par le fait que** les particules de polymères sont présentes à une concentration supérieure à 15 % en poids par rapport au poids total de la composition et la température de transition vitreuse de l'extrait sec de la composition est comprise entre 15 et 35°C et le poids moléculaire moyen de la silicone varie entre 1000 et 1 000 000.

2. Composition selon la revendication 1, **caractérisée par le fait que** ladite dispersion aqueuse résulte de la polymérisation ou de la copolymérisation de monomères choisis parmi le styrène, le butadiène, l'éthylène, le propylène, le vinyl toluène, le vinyl propionate, l'alcool vinylique, l'acrylonitrile, le chloroprène, l'acétate de vinyle, les uréthannes, l'isoprène, l'isobutène, l'éther vinylique, la vinylpyrrolidone, le vinylimidazole et les acides acrylique ou méthacrylique, maléïque, crotonique ou itaconique, leurs esters ou leurs amides.

3. Composition selon l'une quelconques des revendications 1 ou 2, **caractérisée par le fait que** le polymère de la dispersion aqueuse est un polymère comprenant de (a) environ 35 à 75% en poids d'un acrylate d'alkyle, (b) environ 25 à 65% en poids de méthacrylate d'alkyle et (c) environ 1 à 15% en poids d'un ou plusieurs acides carboxyliques éthyléniques ou leurs sels ayant de 3 à 5 atomes de carbone , les radicaux alkyle ayant de 1 à 5 atomes de carbone et ces pourcentages en poids étant exprimés par rapport au poids total du copolymère.

4. Composition selon l'une quelconque des revendications précédentes, **caractérisée en ce que** le polymère est un copolymère acrylate d'éthyle / méthacrylate de méthyle / acide méthacrylique / acide acrylique.

5. Composition selon la revendication 4, **caractérisée en ce que** ledit copolymère comprend de 50 à 60% en poids d'acrylate d'éthyle, de 30 à 40% en poids de méthacrylate de méthyle, de 2 à 10% en poids d'acide acrylique, de 2 à 10% en poids d'acide méthacrylique, la concentration totale d'acide acrylique et méthacrylique n'excédant pas 15% du poids total du copolymère.

6. Composition selon l'une quelconque des revendications précédentes, **caractérisée par le fait que** la concentration en poids des particules de polymère es comprise entre 15 et 35% par rapport au poids total de la composition.

7. Composition selon l'une quelconque des revendications précédentes, **caractérisée par le fait que** la ou les silicones sont présentes à raison de 0,05 à 10 % en poids par rapport au poids total de la composition.

8. Composition selon la revendication 7, **caractérisée par le fait que** la ou les silicones sont présentes à raison de 0,1 à 3 % en poids par rapport au poids total de la composition.

9. Composition selon l'une quelconque des revendications précédentes, **caractérisées par le fait que** la ou les silicones sont choisies parmi les huiles de silicone, les gommes de silicone et les résines de silicone.

10. Composition selon la revendication 9, **caractérisées par le fait que** la ou les silicones sont choisies parmi les polydialkylsiloxanes ou les polyalkylarylslloxanes, éventuellement modifiés.

11. Compositions selon la revendication 10, **caractérisées par le fait que** la ou les silicones sont choisies parmi les gommes de polydialkylsiloxanes ou de polyalkylarylsiloxane, éventuellement modifiés.

12. Composition selon l'une quelconques des revendications précédentes, **caractérisée par le fait que** les compositions sont des lotions de mise en plis, des lotions pour le brushing.

13. Composition pressurisée en aérosol, **caractérisée par le fait qu'**elle comprend une composition telle que définie à l'une quelconque des revendications précédentes, et au moins un agent propulseur.

14. Composition selon la revendication 13, **caractérisée par le fait que** l'agent propulseur est choisi parmi les hydrocarbures volatils tels que le n-butane, le propane, l'isobutane, le pentane, les hydrocarbures chlorés et/ou fluorés, le gaz carbonique, le protoxyde d'azote, le diméthyléther, l'azote, l'air comprimé et leurs mélanges.

15. Composition selon l'une quelconque des revendications 13 ou 14, **caractérisée par** le fait l'agent propulseur est présent dans des concentrations comprises entre 10 et 50% en poids par rapport au poids total de la composition pressurisée.

16. Composition selon la revendication 15, **caractérisée par** le fait l'agent propulseur est présent dans des concentrations comprises entre 15 et 35% en poids par rapport au poids total de la composition pressurisée.

17. Procédé de traitement cosmétique des matières kératiniques, telles que les cheveux, **caractérisé en ce qu'**il consiste à appliquer sur les matières kératiniques, en particulier par pulvérisation ou vaporisation, une composition cosmétique telle que définie dans l'une quelconque des revendications 1 à 16, puis à effectuer éventuellement un rinçage à l'eau, après un éventuel temps de pose.

18. Utilisation d'une silicone insoluble de poids moléculaire moyen variant entre 1 000 et 1 000 000 pour améliorer la qualité de la vaporisation/pulvérisation d'une composition cosmétique contenant dans un milieu cosmétiquement acceptable aqueux ou hydroalcoolique une dispersion aqueuse de particules insolubles de polymère, la température de transition vitreuse de l'extrait sec de la composition étant comprise entre 15 et 35°C et les particules de polymères étant présentes à une concentration supérieure à 15 % en poids par rapport au poids total de la composition.

## Patentansprüche

1. Kosmetische Zusammensetzung, die in einem kosmetisch akzeptablen, wässrigen oder wässrig-alkoholischen Medium eine wässrige Dispersion von unlöslichen Polymerpartikeln und ein unlösliches Silicon enthält, **dadurch gekennzeichnet, dass** die Polymerpartikel in einer Konzentration über 15 Gew.-%, bezogen auf das Gesamtgewicht der Zusammensetzung, vorliegen, die Glasübergangstemperatur des Trockenextraktes der Zusammensetzung im Bereich von 15 bis 35 °C liegt und die mittlere Molmasse des Silicons im Bereich von 1000 bis 1 000 000 liegt.

2. Zusammensetzung nach Anspruch 1, **dadurch gekennzeichnet, dass** die wässrige Dispersion bei der Polymerisation oder Copolymerisation von Monomeren entsteht, die unter Styrol, Butadien, Ethylen, Propylen, Vinyltoluol, Vinylpropionat, Vinylalkohol, Acrylnitril, Chloropren, Vinylacetat, Urethanen, Isopren, Isobuten, Vinylether, Vinylpyrrolidon, Vinylimidazol und Acrylsäure, Methacrylsäure, Maleinsäure, Crotonsäure oder Itaconsäure und deren Estern oder deren Amiden ausgewählt sind.

3. Zusammensetzung nach einem der Ansprüche 1 oder 2, **dadurch gekennzeichnet, dass** das Polymer der wässrigen Dispersion ein Polymer ist, das (a) etwa 35 bis 75 Gew.-% Alkylacrylat, (b) etwa 25 bis 65 Gew.-% Alkylmethacrylat und (c) etwa 1 bis 15 Gew.-% einer oder mehrerer Carbonsäuren mit ethylenischer Doppelbindung oder ihrer Salze mit 3 bis 5 Kohlenstoffatomen enthält, wobei die Alkylgruppen 1 bis 5 Kohlenstoffatome aufweisen und die Mengenangaben in Gewichtsprozent, bezogen auf das Gesamtgewicht des Copolymers, ausgedrückt sind.

4. Zusammensetzung nach einem der vorhergehenden Ansprüche, **dadurch gekennzeichnet, dass** das Polymer ein Ethylacrylat/Methylmethacrylat/Methacrylsäure/Acrylsäure-Copolymer ist.

5. Zusammensetzung nach Anspruch 4, **dadurch gekennzeichnet, dass** das Copolymer 50 bis 60 Gew.-% Ethylacrylat, 30 bis 40 Gew.-% Methylmethacrylat, 2 bis 10 Gew.-% Acrylsäure und 2 bis 10 Gew.-% Methacrylsäure enthält, wobei die Gesamtkonzentration von Acrylsäure und Methacrylsäure 15 % des Gesamtgewichts des Copolymers nicht übersteigt.

6. Zusammensetzung nach einem der vorhergehenden Ansprüche, **dadurch gekennzeichnet, dass** die Konzentration der Polymerpartikel im Bereich von 15 bis 35 Gew.-%, bezogen auf das Gesamtgewicht der Zusammensetzung, liegt.

7. Zusammensetzung nach einem der vorhergehenden Ansprüche, **dadurch gekennzeichnet, dass** das Silicon oder die Silicone in Mengenanteilen von 0,05 bis 10 Gew.-%, bezogen auf das Gesamtgewicht der Zusammensetzung, vorliegen.

8. Zusammensetzung nach Anspruch 7, **dadurch gekennzeichnet, dass** das Silicon oder die Silicone in Mengenanteilen von 0,1 bis 3 Gew.-%, bezogen auf das Gesamtgewicht der Zusammensetzung, vorliegen.

9. Zusammensetzung nach einem der vorhergehenden Ansprüche, **dadurch gekennzeichnet, dass** das Silicon oder die Silicone unter den Siliconölen, Silicongummis und Siliconharzen ausgewählt sind.

10. Zusammensetzung nach Anspruch 9, **dadurch gekennzeichnet, dass** das Silicon oder die Silicone unter den Polydialkylsiloxanen oder Polyalkylarylsiloxanen, die gegebenenfalls modifiziert sind, ausgewählt sind.

11. Zusammensetzung nach Anspruch 10, **dadurch gekennzeichnet, dass** das Silicon oder die Silicone unter den Polydialkylsiloxangummis oder Polyalkylarylsiloxangummis, die gegebenenfalls modifiziert sind, ausgewählt sind.

12. Zusammensetzung nach einem der vorhergehenden Ansprüche, **dadurch gekennzeichnet, dass** es sich bei den Zusammensetzungen um Lotionen für Wasserwellen und Lotionen zum Fönen handelt.

13. Als Aerosol unter Druck gesetzte Zusammensetzung, **dadurch gekennzeichnet, dass** sie eine Zusammensetzung nach einem der vorhergehenden Ansprüche und mindestens ein Treibmittel enthält.

14. Zusammensetzung nach Anspruch 13, **dadurch gekennzeichnet, dass** das Treibmittel unter den flüchtigen Kohlenwasserstoffen, wie n-Butan, Propan, Isobutan, Pentan, chlorierten und/oder fluorierten Kohlenwasserstoffen, Kohlendioxid, Distickstoffoxid, Dimethylether, Stickstoff, Druckluft und deren Gemischen ausgewählt ist.

15. Zusammensetzung nach einem der Ansprüche 13 oder 14, **dadurch gekennzeichnet, dass** das Treibmittel in Konzentrationen von 10 bis 50 Gew.-%, bezogen auf das Gesamtgewicht der unter Druck gesetzten Zusammensetzung, vorliegt.

16. Zusammensetzung nach Anspruch 15, **dadurch gekennzeichnet, dass** das Treibmittel in Konzentrationen von 15 bis 35 Gew.-%, bezogen auf das Gesamtgewicht der unter Druck gesetzten Zusammensetzung, vorliegt.

17. Verfahren zur kosmetischen Behandlung von Keratinsubstanzen, wie dem Haar, **dadurch gekennzeichnet, dass** es darin besteht, auf die Keratinsubstanzen insbesondere durch Zerstäuben oder Vernebeln eine kosmetische Zusammensetzung nach einem der Ansprüche 1 bis 16 aufzubringen und dann gegebenenfalls nach einer Einwirkzeit gegebenenfalls mit Wasser zu spülen.

18. Verwendung eines unlöslichen Silicons mit einer mittleren Molmasse von 1000 bis 1 000 000 zur Verbesserung der Güte der Zerstäubung/Vernebelung einer kosmetischen Zusammensetzung, die in einem kosmetisch akzeptablen, wässrigen oder wässrig-alkoholischen Medium eine wässrige Dispersion von unlöslichen Polymerpartikeln enthält, wobei die Glasübergangstemperatur des Trockenextrakts der Zusammensetzung im Bereich von 15 bis 35 °C liegt und die Polymerpartikel in einer Konzentration über 15 Gew.-%, bezogen auf das Gesamtgewicht der Zusammensetzung, vorliegen.

## Claims

1. Cosmetic composition comprising, in a cosmetically acceptable aqueous or aqueous/alcoholic medium, an aqueous dispersion of insoluble polymer particles and an insoluble silicone, **characterized in that** the polymer particles are present at a concentration of greater than 15% by weight with respect to the total weight of the composition, the glass transition temperature of the dry matter of the composition is between 15 and 35°C and the average molecular weight of the silicone varies between 1 000 and 1 000 000.

2. Composition according to Claim 1, **characterized in that** the said aqueous dispersion results from the polymerization or from the copolymerization of monomers chosen from styrene, butadiene, ethylene, propylene, vinyltoluene, vinyl propionate, vinyl alcohol, acrylonitrile, chloroprene, vinyl acetate, urethanes, isoprene, isobutene, vinyl ether, vinylpyrrolidone, vinylimidazole and acrylic or methacrylic, maleic, crotonic or itaconic acids, their esters or their amides.

3. Composition according to either one of Claims 1 and 2, **characterized in that** the polymer of the aqueous dispersion is a polymer comprising from (a) approximately 35 to 75% by weight of an alkyl acrylate, (b) approximately 25 to 65% by weight of alkyl methacrylate and (c) approximately 1 to 15% by weight of one or more ethylenic carboxylic acids or their salts having from 3 to 5 carbon atoms, the alkyl radicals having from 1 to 5 carbon atoms and these percentages by weight being expressed with respect to the total weight of the copolymer.

4. Composition according to any one of the preceding claims, **characterized in that** the polymer is an ethyl acrylate/methyl methacrylate/methacrylic acid/acrylic acid copolymer.

5. Composition according to Claim 4, **characterized in that** the said copolymer comprises from 50 to 60% by weight of ethyl acrylate, from 30 to 40% by weight of methyl methacrylate, from 2 to 10% by weight of acrylic acid and from 2 to 10% by weight of methacrylic acid, the total concentration of acrylic and methacrylic acid not exceeding 15% of the total weight of the copolymer.

6. Composition according to any one of the preceding claims, **characterized in that** the concentration by weight of the polymer particles is between 15 and 35% with respect to the total weight of the composition.

7. Composition according to any one of the preceding claims, **characterized in that** the silicone or silicones are present in the proportion of 0.05 to 10% by weight with respect to the total weight of the composition.

8. Composition according to Claim 7, **characterized in that** the silicone or silicones are present in the proportion of 0.1 to 3% by weight with respect to the total weight of the composition.

9. Composition according to any one of the preceding claims, **characterized in that** the silicone or silicones are chosen from silicone oils, silicone gums and silicone resins.

10. Composition according to Claim 9, **characterized in that** the silicone or silicones are chosen from optionally modified polydialkylsiloxanes or polyalkylarylsiloxanes.

11. Compositions according to Claim 10, **characterized in that** the silicone or silicones are chosen from optionally modified polydialkylsiloxane or polyalkylarylsiloxane gums.

12. Composition according to any one of the preceding claims, **characterized in that** the compositions are hairsetting lotions or blow-drying lotions.

13. Composition pressurized in an aerosol, **characterized in that** it comprises a composition as defined in any one of the preceding claims and at least one propellant.

14. Composition according to Claim 13, **characterized in that** the propellant is chosen from volatile hydrocarbons, such as n-butane, propane, isobutane, pentane or chlorinated and/or fluorinated hydrocarbons, carbon dioxide gas, nitrous oxide, dimethyl ether, nitrogen, compressed air and their mixtures.

15. Composition according to either one of Claims 13 or 14, **characterized in that** the propellant is present in concentrations of between 10 and 50% by weight with respect to the total weight of the pressurized composition.

16. Composition according to Claim 15, **characterized in that** the propellant is present in concentrations of between 15 and 35% by weight with respect to the total weight of the pressurized composition.

17. Process for the cosmetic treatment of keratinous substances, such as hair, **characterized in that** it consists in applying, to the keratinous substances, in particular by spraying or vaporization, a cosmetic composition as defined in any one of Claims 1 to 16 and in then optionally rinsing with water, optionally after leaving for a period of time.

18. Use of an insoluble silicone with an average molecular weight varying between 1 000 and 1 000 000 for improving the quality of the vaporization/spraying of a cosmetic composition comprising, in a cosmetically acceptable aqueous or aqueous/alcoholic medium, an aqueous dispersion of insoluble polymer particles, the glass transition temperature of the dry matter of the composition being between 15 and 35°C and the polymer particles being present at a concentration of greater than 15% by weight with respect to the total weight of the composition.
